# EUROPEAN PATENT APPLICATION

(11) **EP 2 055 699 A1**
(43) Date of publication of application: **06.05.2009**
(21) Application number: 07425687.6
(22) Date of filing: 30.10.2007
(51) Int. Cl.: C07D 317/36, C07D 317/38

(54) **Process for producing cyclic carbonates**

(71) Applicant: Enel Produzione S.p.A., 00198 Roma RM (IT)
(72) Inventor: Politi, Monia c/o ENEL GEM AT Ricerca, 72020 Tuturano (Brindisi) (IT); Calo', Vincenzo c/o Chemistry Department, 70126 Bari (IT); Nacci, Angelo c/o Chemistry Department, 70126 Bari (IT)
(74) Representative: Ferraiolo, Ruggero

(57) **Abstract**

A mixture of catalysts composed of tetrabutylammonium iodide (TBAI) and zinc iodide (ZnI2) is used for producing cyclic carbonates, in particular variously substituted 2-oxo-1,3-dioxolanes, starting from an epoxy and carbon dioxide, under conditions of pressure and temperature approaching ambient values and in the absence of a solvent.

## Description

### Field of the invention

The present invention regards a process for producing cyclic carbonates of the general formula given in diagram 1. The starting materials used in this process are the corresponding epoxies and gaseous carbon dioxide.

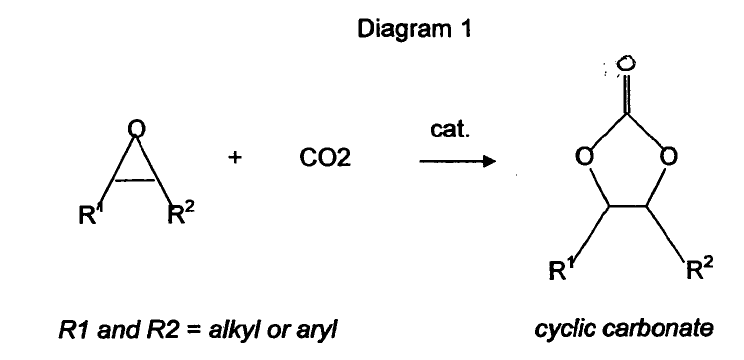

The cyclic carbonates (2-oxo-1,3-dioxolanes) are important industrial intermediates offering a variety of applications. They are in fact excellent dipolar aprotic solvents, electrolytes for batteries, precursors for the synthesis of polycarbonates and other polymeric materials, as well as intermediates for the preparation of pharmaceutical and fine chemical products.

### State of the art

The synthesis of cyclic carbonates most widespread on an industrial scale is realized by a reaction between an appropriate epoxy and carbon dioxide (diagram 1). This process is of extreme interest in the field of green chemistry, as it allows trapping an environmentally harmful gas such as carbon dioxide (the main agent responsible for the hothouse effect) in widely used products such as cyclic carbonates.

A vast range of catalysts, both homogeneous and heterogeneous, has been studied to promote this process. Important results were achieved using alkaline metal halides, organic bases, metallic oxides, zeolites, titanium silicates, smectites and metallic complexes (J. Organomet. Chem., 2005, 3490-3497).

Particulary effective are bifunctional catalysts constituted of electrophilic-nucleophilic binary mixtures, wherein the first component is usually a Lewis acid, whose purpose is facilitate the opening of the oxyranic ring by coordinating the oxygen atom, while the second component is a species with a large number of electrons (usually an anion) whose assigned purpose is to open the epoxy ring by a nucleophilic attack.

The main constituents of these systems include, among others, salts or metal complexes (mostly of Zn, Al, Ni, Sn and Mg) forming the electrophile portion, while the nucleophile counterpart is formed by imidazole, phosphonium and quaternary ammonium salts carrying a halide as a counterion.

The Tetrahedron Lett., 45, 8307/8310 (2004) describes the use of a mixture of ZnCl₂ and 1-butyl-3-methylimidazole bromide as a catalytic system for carbonating a series of mono-substituted epoxies run at 100°C and a pressure of 1.5 MPa (ab. 14.8 at). The catalyst can be recycled six times. The same process is described in the Bulletin of the Korean Chemical Society, 23(7), 1027-1028 (2002), where the substitution of the Cl⁻ and Br⁻ ions of the subject mixture with the iodide ion (ZnCl₂ plus 1-butyl-3-methylimidazole iodide) allows operating at lower temperatures (40°C).

The Journal of Molecular Catalysis A: Chemical, 210, 31-34 (2004) describes on the other hand a catalytic system composed of an aluminium (III) complex carrying as a binder a Shiff base [a complex known as (Salen)AlCl] conjugated with a quaternary ammonium salt. The reaction occurs only on mono-substituted epoxies at ambient temperature and a CO₂-pressure of 0.6 MPa (ab. 5.9 at).

The Chem. Commun. (16), 2042-2043 (2003) describes the carbonating of terminal epoxies promoted by a nickel (II) [(PPh₃)₂NiCl₂)] complex in the presence of powdered zinc and Bu₄NBr. At any rate, the reaction demands drastic conditions such as a CO₂-pressure of 2.5 MPa and a temperature of 120°C.

Finally, the Bulletin of the Chemical Society of Japan 60, 1152-4 (1987) describes the use of a mixture composed of an organostannate and a quaternary phosphonium salt (Bu₃Snl-Bu₄Pl) at high pressures, while the only case of a usage at moderate conditions (P and T close to ambient values) is reported by Chemische Berichte, 119(3), 1090-4 (1986), while using a mixture of zinc chloride and tetrabutyl-ammoniumbromide (ZnCl₂/Bu₄NBr).

In the description to follow, we conventionally intend moderate conditions to be ab. 1 atm and temperatures in the range from -10 to 70°C.

The interest toward preparing cyclic carbonates is otherwise witnessed by an abundant patent literature. Refer to the following documents.

The WO 84/00371 describes the reaction in the presence of triphenylphosphine as a catalyst, while operating at a pressure of 21 bar and using an alcohol as a solvent.

The US 3,748,345 reports the use of phosphinic nickel complexes as catalysts, at a pressure of 35 bar, to yield a 50% conversion of the epoxy to a cyclic carbonate, while on the other hand the EP 0 212 409 and US 4,892,954 use amines, ammonium salts, phosphines and co-catalysts to boost reaction speed.

The US 5,153,333 describes the use of phosphonium salts as catalysts, temperatures in the range of 60 to 200°C and pressures between 1 and 5 atm.

The RU 2128658 describes the use of cobalt halides as catalysts, temperatures in the range of 130 to 150°C and dimethylformamide as a solvent.

A series of patent documents also reports the use of bifunctional catalysts, such as for instance the CN 1817877 and CN 1817878, which use tetraalkylammonium and/or phosphonium salts conjugated with Zn, Fe, Cr and Ni-salts, while operating under drastic conditions (0.1-5.0 MPa and 40-210°C), or the CN 1696123, which uses Zn and Fe-salts (Znl2, ZnCl2, ZnBr2 or FeBr3) in the presence of a guanidine salt at 100-150°C and 1.5-5 MPa.

Moreover, the CN 1566110 and CN 1566111 describe the carbonation of propylene oxide in the presence of complexes of (II) [(PPh₃)₂NiCl₂], powdered zinc and quaternary ammonium salts at 1.5-2.5 MPa and 50-150°C.

The CN 1343668 also describes a carbonation of the epoxies in ionic liquids in the presence of additives such as alkaline metal halides and quaternary ammonium salts at 100-140°C and 1.5-4.5 MPa. Lastly, the US 2002013477, US 6407264 and KR 2001081225 are among the few patents that also describe the reaction between disubstituted epoxies and CO2 catalyzed by mixtures of metallic halides and pyridine.

### Drawbacks of the state of the art

Each of the synthesis processes described above presents drawbacks. The most significant among these are described as follows:
- Drastic operating conditions. The larger part of the mentioned catalytic systems operates at high temperatures and/or pressures, while an efficient catalytic system must be active at temperatures and pressures approaching ambient conditions.
- Use of toxic or volatile solvents. Many of the methods outlined above use volatile and/or toxic organic solvents such as dimethylformamide, aromatic hydrocarbons or chlorinated compounds (these solvents are frequently designated as VOCs, or "Volatile Organic Compounds"), which are difficult to recycle and demand elaborate and costly separating and disposing procedures.
- Poor catalyst turnover. Most of the methods described offer very scant data on reutilizing the catalyst system, which seems to degrade with advancing cycles. On the contrary, a catalytic system for large-scale application must allow prolonged and possibly unlimited re-use with high "turnover number" (TON) values, a total absence of byproducts to be disposed of, and a lack of contamination of the final product.

### Abstract of the invention

The scope of the present invention is to provide a synthesis of cyclic carbonates at moderate pressure and temperature conditions, so as to eliminate the mentioned drawbacks and furnish said products at high yields.

Experimental investigations have allowed identifying in zinc iodide (Znl₂) and tetrabutylammonium iodide (Bu₄Nl) a bifunctional catalytic system that achieves the mentioned scope and provides advantages that will be outlined in greater detail in the following description.

As amply illustrated in the foregoing bibliographical notes and in the patent literature, despite the fact that these two salts have used individually in previous inventions for processes having the same scope, their combined usage constitutes a novelty that introduces substantial improvements, as it demands only very moderate operating conditions and offers the opportunity of using a vast range of epoxy substrates. In this regard, the new catalytic system reacts with those disubstituted epoxy substrates (both R1 and R2 in diagram 1 are other than hydrogen) that turn out to be inert or scarcely applicable in the large majority of catalytic systems conforming to the state of the art.

The following diagram offers a comparison between the efficacy of the three tetrabutylammonium halides [tetrabutylammonium chloride (TBAC), tetrabutylammonium bromide (TBAB) and tetrabutylammonium iodide (TBAI) as co-catalysts of zinc iodide in carbonating the 1-butene oxide, run at ambient pressure and temperature.

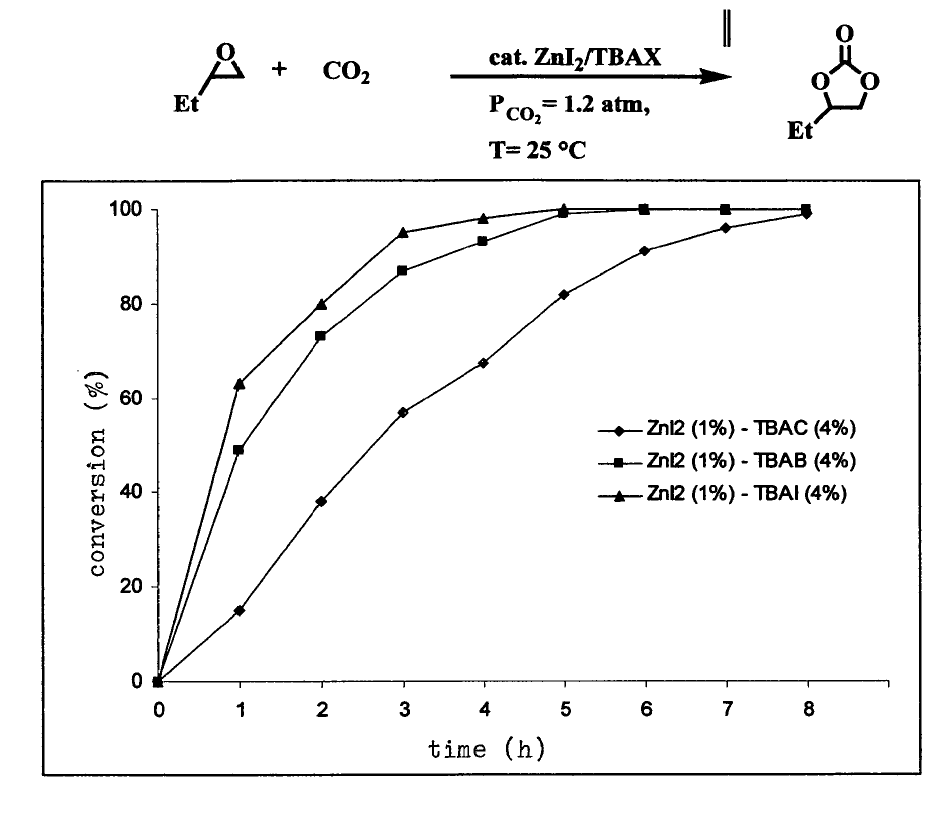

The diagram clearly shows that the maximum catalytic activity is precisely obtain with the pair Znl₂/TBAI. The method comprises the following phases:
- i) Inducing a reaction between carbon dioxide (CO2) and an epoxy of the general formula (II) 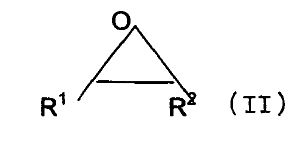 Wherein R¹ and R² can be equal to H, an alkyl (from C1 to C4), aryl, alcoximethyl and aryloximethyl, at ambient or next to ambient temperature, in the presence of a mixture of zinc iodide (Znl₂) and tetrabutylammonium iodide (Bu₄Nl):
- ii) Isolating the 2-oxo-1,3-dioxolane from the mixture of the two catalysts obtained by the previous reaction.

The two catalysts' capacity of maintaining their catalyzing action after the first isolation of the product was also tested in subsequent cycles of synthesizing the cyclic carbonates, and this capacity was found to exist for an unlimited number of cycles.

Moreover, it was found that for securing conversions in short time periods (4-8 hours), the minimum percentages of the two co-catalysts Znl₂/TBAI were 1% for the zinc iodide and 4% of the tetrabutylammonium iodide, respectively. The relative 1:4 ratio of the salts is optimized to achieve a maximum efficiency of the catalytic system.

The process is actuated at temperatures in the range from -10°C to 70°C, preferably at an operating CO₂ pressure of ab. 1 atm.

### Advantages of the invention

The principal advantages of the present process are that they supply a high yield of cyclic carbonates at moderate conditions of pressure and temperature. In addition:
- The groups R1 and R2 can be selected independently from each other and can also be an alkyl or aryl radical. The conversion of the epoxy (II) into a cyclic carbonate (I) is generally quantitative and does not produce byproducts deriving from the polymerization of the epoxy (II) or the degradation of the catalysts.
- The CO2 operating pressure is 1.2 atm, a value optimized for obtaining a complete conversion of the epoxy in 4 hours, while if operating at atmospheric pressure (1.0 atm) the reaction comes to completion in 15-20 hours. In certain cases the reaction is already completed after one hour.
- The operating temperature varies in the range from -10 to 70°C depending on the degree of epoxy substitution. In particular, for the mono-substituted epoxies (at least one of the two groups R¹ or R² equals hydrogen) the process is exothermic, and in order to prevent dangerous overpressures, the volatile epoxies require a cooling of the mixture at least during the first reaction hour. For disubstituted epoxies such as the 2,3-butene oxide, the optimum working temperature is 50°C.
- The isolation of the 2-oxo-1,3-dioxolanes may be conducted in accordance with the methods known to the specialist in the trade. Because the operation is run in the absence of a solvent and the catalysts are non-volatile salts, a vacuum-distillation is the most favourable technique. The present invention optimized the vacuum-distillation method for the most volatile products (R¹ = H, CH₃, CH₂CH₃, R² = H, CH₃). This procedure allows a complete recovery of the catalytic system, which can operate with a high number of successive reactions. As an example, refer to the following diagram, which describes the recycling of the catalytic system Znl₂/Bu₄Nl (1% / 4%) for carbonating the 1-butene oxide. In performing the vacuum-distillation of the product, 10 reaction cycles can be run while leaving the catalytic system's efficiency unchanged, as can be gathered from the constancy of the reaction periods (always of 4 hours) and from the values of the isolated yields, which are always above 95% (TON = ab. 1,000).

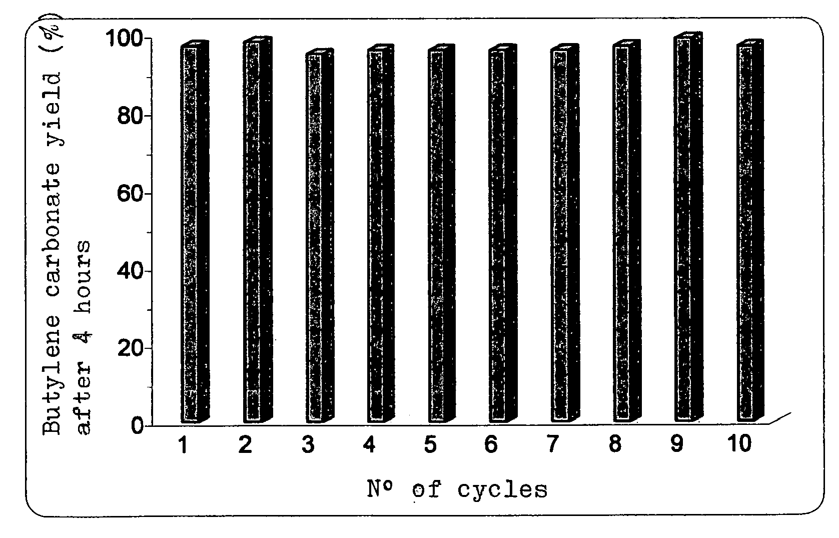

The 2-oxo-1,3-dioxolanes may alternatively be separated by extracting them with solvents, in which the catalysts Znl₂ and TBAI are insoluble, such as for instance ethyl-ether or ciclohexane.

Other advantages of the invention are herewith summarized as follows.

Environmental compatibility: the reaction fully respects a series of key principles characterizing an environmentally compatible process, such as for instance the principle of "atom economy" and of "energy demand" (which privileges processes run at ambient pressure and temperature).

Products of high added value: the method is particularly advantageous for the simplest epoxies (ethylene, propylene, and butylenes oxides) for two main reasons: i) they provide the cyclic carbonates with the highest CO₂-valorizing coefficients and ii) being liquid, they allow applying the simplest procedure, meaning in the absence of a (bulk) solvent. It is understood that by appropriate modifications, such as for instance by using an ionic liquid as a solvent (for instance 1-butyl-3-methylimidazole chloride) and/or by running the extraction with an organic solvent for non-volatile products, the procedure is applicable to any epoxy.

Efficiency of the catalytic system: the catalytic system used proves to be very active and robust, being capable of withstanding innumerable recycling operations envisioning a heating up to 80°C (the TON is estimated at values exceeding 10.000). The system is bifunctional, as it is constituted of the combination of a nucleophile (TBAI) and an electrophile element (Znl₂). The two catalysts operate with a potent synergy, as evidenced by the weak activity exhibited by the two catalysts when operating individually.

Operating simplicity: this is certainly one of the prime advantages of this process, whose truth can be realized based on the following observations:
- no particular apparatuses or reactors (pressure vessels) are needed, the use of a glass flask with a single, minimally sealing neck being adequate;
- no pretreating of the catalysts is needed. It should be noted that both the epoxy and the catalysts are low-cost commercial products; the economical advantage is further boosted by the absence of any preliminary preparations o activations of the catalytically active species;
- the reaction primes itself spontaneously after connecting the reactor to the pressure cylinder while maintaining a slight CO2-overpressure (ab. 1.2 atm) and vigorous agitation;
- the operating simplicity is also extended to a monitoring of the epoxy conversion to the carbonate, which can be performed by a simple weight increase of the reaction mixture;
- the reaction product can be obtained with a high yield and purity after vacuum distillation, and the catalyst can be recycled innumerable times.

### Detailed description of the invention

The invention is described in the following through examples of producing cyclic carbonates, which are non-limiting as regards the ways of realizing the invention.

### EXAMPLE 1

2-oxo-1,3-dioxolane (ethylene carbonate)

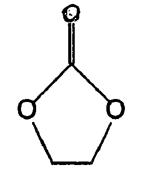

| Reagents/solvents/catalysts | PM g/moles | Mmoles | Grams (or ml) |
|---|---|---|---|
| Ethylene oxide (d = 0.882 g/ml) | 44.05 | 172 | 10.01 (12 ml) |
| TBAI | 369.38 | 13.8 (8%) | 5.09 g |
| Znl₂ | 319.20 | 3.44 (2%) | 1.10 g |
| P_{CO2} | 1.2 atm | | |
| Temperature | - 10°C | | |
| Time | 4 hours | | |

In a 100-ml Schlenk (see the following diagram) fitted with a magnetic stirrer, the Bu₄Nl and Znl₂ are introduced in the given quantities. After conditioning the Schlenk with a CO₂-atmosphere (three cycles of vacuum/CO₂) the system is connected, through a tygon tube, to the ethylene oxide containing cylinder, which is held upside down and at a height above that of the Schlenk, so as to ease the liquid transfer by gravity.

The Schlenk is cooled in a bath at -10°C, connected to the CO₂ line and kept under agitation at the operating pressure of 1.2 atm for the time needed. As the reaction proceeds, the mixture turns ever more viscous, up to becoming solid at the end of the process (the ethylene carbonate is in fact solid at ambient temperature). The mixture is transferred to the appropriate apparatus and subjected to vacuum distillation. The ethylene carbonate obtained at a high degree of purity quickly solidifies in the distillate collecting container (Tₘₑₗₜ = 35-38°C, T_{boil} = 46°C at the pressure of 2*10⁻³ mbar).

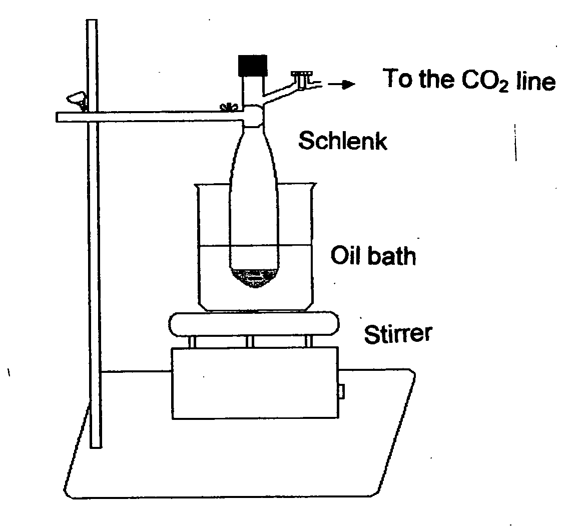

### EXAMPLE 2

4-methyl-2-oxo-1,3-dioxolane (propylene carbonate)

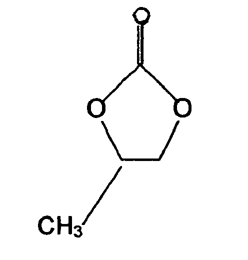

| Reagents/solvents/catalysts | PM g/moles | Mmoles | Grams (or ml) |
|---|---|---|---|
| Propylene oxide (d ≈ 0.829 g/ml) | 58.08 | 172 | 10.01 (12 ml) |
| TBAI | 369.38 | 6.90 (4%) | 2.55 g |
| Znl₂ | 319.20 | 1.72 (2%) | 0.55 g |
| P_{CO2} | 1.2 atm | | |
| Temperature | ambient | | |
| Time | 4 hours | | |

In a 50 ml Schlenk equipped with a magnetic stirrer, the epoxy, TBAI and Znl2 are introduced in the given quantities. The two salts are non-soluble in the epoxy and thus remain in suspension. The reactor is hermetically closed and the operation is performed using a number of vacuum and CO₂ cycles. The reactor is then connected to the CO2 line, while keeping it agitated at the given pressure and ambient temperature for the time needed.

The vacuum distillation (maximum bath temperature 85°C) allows obtaining the propylene carbonate, with a high yield (92%) and a purity exceeding 99% (glc-purity), as a colorless, pungent odour liquid (T_{boil} = 48°C at the pressure of 2*10⁻³ mbar).

### EXAMPLE 3

4-ethyl-2-oxo-1,3-dioxolane (1-butene carbonate)

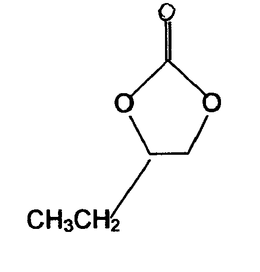

| Reagents/solvents/catalysts | PM g/moles | Mmoles | Grams (or ml) |
|---|---|---|---|
| 1-butene oxide (d ≈ 0.829 g/ml) | 72.11 | 172 | 12.44 (15 ml) |
| TBAI | 369.38 | 6,90 (4%) | 2.55 g |
| Znl₂ | 319.20 | 1.72(1%) | 0.55 g |
| P_{CO2} | 1.2 atm | | |
| Temperature | ambient | | |
| Time | 4 hours | | |

When operating as described in Example 2, the product is obtained with a yield of 97% (T_{boil} = 80°C at the pressure of 2 mm Hg).

### EXAMPLE 4

4,5-dimethyl-1,3-dioxolane-2-one (2-butene carbonate)

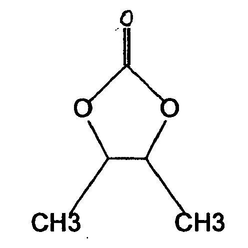

| Reagents/solvents/catalysts | PM g/moles | Mmoles | Grams (or ml) |
|---|---|---|---|
| 2-butene oxide (d = 0.804 g/ml) | 72.11 | 22.1 | 1.61 (2 ml) |
| TBAI | 369.38 | 0.884 (4%) | 0.326 g |
| Znl₂ | 319.20 | 0.442 (2%) | 0.141 g |
| P_{CO2} | 1.5 atm | | |
| Temperature | 50°C | | |
| Time | 48 hours | | |

When operating as described in Example 2, the product is obtained with a yield of 91 % (T_{boil} = 50°C at the pressure of 0.2 mm Hg).

### EXAMPLE 5

4-phenyl-2-oxo-1,3-dioxolane (styrene carbonate)

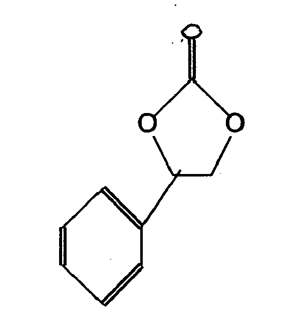

| Reagents/solvents/catalysts | PM g/moles | Mmoles | Grams (or ml) |
|---|---|---|---|
| Styrene oxide (d ≈ 1.054 g/ml) | 120.15 | 87.72 | 10.54 (10 ml) |
| TBAI | 369.38 | 3.51 (4%) | 1.30 g |
| Znl₂ | 319.20 | 0.877 (1%) | 0.28 g |
| P_{CO2} | 1.2 atm | | |
| Temperature | ambient | | |
| Time | 14 hours | | |

The reaction is run as in Example 2. However, because the styrene carbonate is a high boiling point liquid (T_{boil} = 251 °C), the purification of the product is performed in a different manner. The reaction mixture is in this case dissolved in 100 ml of ethyl-ether, in which the two catalysts (TBAI and Znl₂) are insoluble. After filtering, the two mother solutions are separated from the ethyl-ether by evaporation, to produce the product with a yield of 92% and a purity of 98% (glc).

### EXAMPLE 6

Hexahydro-benzo[1,3]dioxol-2-one

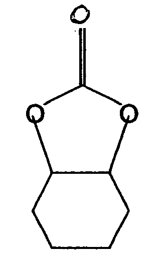

| Reagents/solvents/catalysts | PM g/moles | Mmoles | Grams (or ml) |
|---|---|---|---|
| Cyclohexen-oxide (d = 0.970 g/ml) | 98.15 | 98.8 | 9.7 (10 ml) |
| TBAI | 369.38 | 9.88 (10%) | 3.65 g |
| Znl₂ | 319.20 | 4.94 (5%) | 1.58 g |
| P_{CO2} | 1.2 atm | | |
| Temperature | 60°C | | |
| Time | 14 hours | | |

The reaction and method of isolating the product are as in Example 5. The evaporation of the ethyl-ether from the filtrate supplies the product at a yield of 90% and a purity of 98% (glc).

### EXAMPLE 7

4-(isopropoximethyl)-2-oxo-1,3-dioxolane

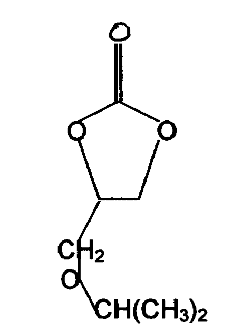

| Reagents/solvents/catalysts | PM g/moles | Mmoles | Grams (or ml) |
|---|---|---|---|
| Glycidil-isopropyl-ether (d = 0.919 g/ml) | 116.16 | 79.1 | 9.19 g (10 ml) |
| TBAI | 369.38 | 3.16 (4%) | 1.16 g |
| Znl₂ | 319.20 | 0.79 (1%) | 0.25 g |
| P_{CO2} | 1.2 atm | | |
| Temperature | ambient | | |
| Time | 14 hours | | |

When operating as described in Example 5, the product is obtained with a yield of 98%.

### EXAMPLE 8

4-(phenoximethyl)-2-oxo-1,3-dioxolane

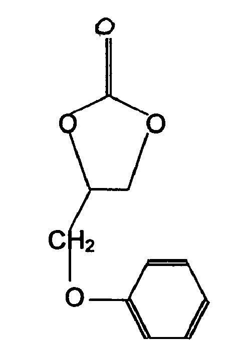

| Reagents/solvents/catalysts | PM g/moles | Mmoles | Grams (or ml) |
|---|---|---|---|
| Glycidil-phenyl-ether (d ≈ 1.107 g/ml) | 150.18 | 73.7 | 11.07 (10ml) |
| TBAI | 369.38 | 2.95 (4%) | 1.09 g |
| Znl₂ | 319.20 | 0.737 (1%) | 0.235 g |
| P_{CO2} | 1.2 atm | | |
| Temperature | ambient | | |
| Time | 14 hours | | |

When operating as described in Example 5, the product is obtained with a yield of 92%.

### EXAMPLE 9

4-(4-nonyl-phenoximethyl)-2-oxo-1,3-dioxolane

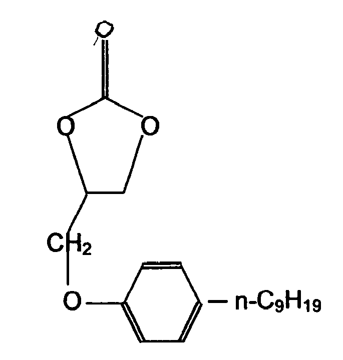

| Reagents/solvents/catalysts | PM g/moles | Mmoles | Grams (or ml) |
|---|---|---|---|
| Glycidil-4-nonylphenyl-ether(d≈ 0.978g/ml) | 276.41 | 35.4 | 9.78 g (10 ml) |
| TBAI | 369.38 | 1.416 (4%) | 0.523 g |
| Znl₂ | 319.20 | 0.354 (1%) | 0.112 g |
| P_{CO2} | 1.2 atm | | |
| Temperature | ambient | | |
| Time | 14 hours | | |

When operating as described in Example 5, the product is obtained with a yield of 90%.

### EXAMPLE 10

4-butyl-2-oxo-1,3-dioxolane (1-hexen-carbonate)

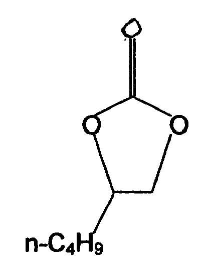

| Reagents/solvents/catalysts | PM g/moles | Mmoles | Grams (or ml) |
|---|---|---|---|
| 1-hexen-oxide (d= 0.833 g/ml) | 100.16 | 83.2 | 8.33 g (10 ml) |
| TBAI | 369.38 | 3.328 (4%) | 1.23 g |
| Znl₂ | 319.20 | 0.832 (1%) | 0.265 g |
| P_{CO2} | 1.2 atm | | |
| Temperature | ambient | | |
| Time | 4 hours | | |

When operating as described in Example 5, the product is obtained with a yield of 96%.

## Claims

1. Process for producing cyclic carbonates such as substituted 2-oxo-1,3-dioxolanes of the formula 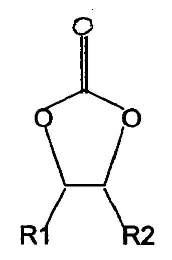 **characterized in that** it includes the phases of:
i) reacting an epoxy of the general formula 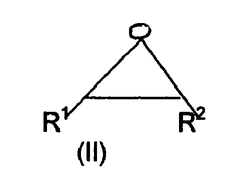 with carbon dioxide (CO₂) in the presence of a mixture of the two catalysts zinc iodide (Znl₂) and tetrabutylammonium-iodide (Bu₄Nl), where the reaction produces a mixture of 2-oxo-1,3-dioxolane and of the two catalysts;
ii) isolating the mixture of the two catalysts with the 2-oxo-1,3 dioxolane obtained from the previous reaction, by a vacuum distillation.

2. Process for producing cyclic carbonates such as substituted 2-oxo-1,3-dioxolanes of the formula 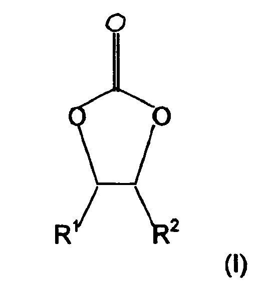 **characterized in that** it includes the phases of:
i) reacting an epoxy of the general formula 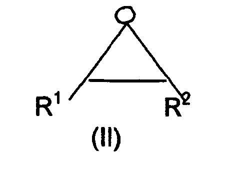 with carbon dioxide (CO₂) in the presence of a mixture of the two catalysts zinc iodide (Znl₂) and tetrabutylammonium-iodide (Bu₄Nl), where the reaction produces a mixture of 2-oxo-1,3-dioxolane and the two catalysts;
ii) isolating from the mixture of the two catalysts the 2-oxo-1,3 dioxolane by dissolving in ethyl-ether and subsequently filtering off and evaporating the ethyl-ether, where the cyclic carbonate thus obtained has a boiling temperature above 250°C.

3. Process according to claim 1 or claim 2, **characterized in that** the first phase i) of the process occurs in a time of between 4 and 8 hours when the mixture of the catalysts comprises 1% of Znl₂ and 4% of TBAI, in moles, with respect to the epoxy.

4. Process according to claim 1 or claim 2, **characterized in that** the reaction temperature is between -10°C and 70°C and the CO₂-pressure is about 1 atm.

5. Process according to claim 1 or claim 2, **characterized in that** R² = H and R¹ is chosen among H, CH₃ and CH₂CH₃, and further that R¹ = R² = CH₃.

6. Process according to claim 1 or claim 2, **characterized in that** R² = H and R¹ is exactly butyl or phenyl or R'-O-CH₂-, where R' is chosen among phenyl, isopropyl, and 4-nonylphenyl, and further that R¹ = R² = -(CH2)₄₋.

7. Process according to the previous claims, **characterized in that** the mixture of the two catalysts maintains its catalyzing action for an unlimited number of cycles after the first isolation of the product.
